# EUROPEAN PATENT APPLICATION

(11) **EP 4 806 889 A1**
(43) Date of publication of application: **16.09.2026**
(21) Application number: 25162890.5
(22) Date of filing: 11.03.2025
(51) Int. Cl.: C07D 307/33

(54) **PROCESS FOR PRODUCING GAMMA-VALEROLACTONE**

(71) Applicant: BASF SE, 67056 Ludwigshafen (DE); BASF Italia S.p.A., 20811 Cesano Maderno (MI) (IT)
(72) Inventor: HIEBER, Gisela, 69120 Heidelberg (DE); IACONO, Giovanni, 00038 Valmontone (IT); HEIDEMANN, Thomas, 67056 Ludwigshafen am Rhein (DE); KUNST, Andreas, 67056 Ludwigshafen am Rhein (DE); SCHREIBER, Michael, 67056 Ludwigshafen am Rhein (DE); MÜLLER, Christian, 67056 Ludwigshafen am Rhein (DE)
(74) Representative: Meissner Bolte Partnerschaft mbB

(57) **Abstract**

The present invention relates to a process for the production of gamma-valerolactone, which comprises a hydrogenation of an ethyl levulinate with hydrogen in the presence of a heterogeneous ruthenium catalyst, which comprises elemental ruthenium supported on activated carbon carrier.

## Description

The present invention relates to a process for producing gamma-valerolactone, which comprises a hydrogenation of ethyl levulinate in the presence of a heterogeneous ruthenium catalyst.

Gamma-valerolactone (GVL), also referred to as 5-methyloxolan-2-on, is a valuable organic compound that can be used for numerous purposes. For example, it is a promising substitute for potentially problematic solvents, such as N-methyl pyrrolidone and dimethylformamide, and can be used for the production of adipic acid. GVL can be produced from levulinic acid which itself can be obtained biological feedstock. GVL was identified as a potential "green fuel", as it retains 97% of the energy of glucose and can be blended by itself in gasoline where it performs comparably to conventional ethanol/gasoline mixtures. Morover, GVL has been suggested as a solvent in the thermocatalytic production of soluble carbohydrates from lignocellulosic materials, such as corn stover and wood at high yields.

WO 02/074760 desribes the production of GVL by hydrogenation of levulicinc acid in the presence of a catalytic amount of supported transition metal catalyst having both a hydrogenation and a ring-closing function. The experiments were carried out in dioxan and often resulted in poor selectivity towards GVL and low conversion of levulinic acid, respectively.

EP 2537840 suggests overcomming the disadvantages of poor selectivity towards GVL and low conversion of levulinic acid by carrying out the hydrogenation of levulinic acid in the presence of a solid ruthium catalyst and at least 0.08% b.w. of water relative to the amount of levulinic acid. However, the conversion of levulinic acid by this process is still not satisfactory.

EP 3184517 suggests that the poor selectivity towards GVL can be overcome by hydrogenating levulinic acid under conditions that produce 4-hydroxyvaleric acid followed by conversion of the 4-hydroxyvaleric acid under conditions where further hydrogenation is prevented. The process of requires the hydrogenation of levulinic acid at low temperatures of at most 120°C and a separate reaction step for the conversion 4-hydroxyvaleric to GVL to provide the desired selectivity, which results in low conversion rates and makes the process less economic.

It was surpsingly found that gamma-valerolactone can be produced with high selectivity and high conversion rates directly to GVL by a process which comprises the hydrogenation of ethyl levulinate with hydrogen in the presence of a heterogeneous ruthenium catalyst, which comprises elemental ruthenium supported on an activated carbon carrier.

Therefore, the present invention relates to a process for the production of gamma-valerolactone, which comprises a hydrogenation of ethyl levulinate with hydrogen in the presence of a heterogeneous ruthenium catalyst, which comprises elemental ruthenium supported on activated carbon carrier.

The process of the present invention provides several benefits. First of all, it provides GVL with high conversion rates and high selectivities towards the formation of GVL. In particular conversion rates are much higher than in hydrogenation reactions using ruthenium catalysts containing ruthenium on oxidic carriers, such as zirconium oxide or aluminium oxide. The ruthenium catalysts used in the process of the present invention show no significant loss of activity even after prolonged use and a high selectivity towards the formation of 4-hydroxyvalerates and gamma valerolactone. In particular overreduction to ethyl valerate or 2-methyltetrahydrofurane is minimized or does not occur at all. The process does not necessarily require a second reaction step for the transformation of the intermediately formed ethyl 4-hydroxyvalerate into GVL. The hydrogen of the inventive process can be easily carried out in fixed bed reactors allowing an easy and efficient upscaling to industrial production and high catalyst load. Last but not least, the use of ethyl levulinate turned out to cause less corrosions which is sometimes faced when using a levulinic acid feedstock. In addition, dehydration of the ethanol formed during the cyclization of ethyl 4-hydroxyvalerate to GVL does not occur to any significant extent, so that it can be recovered, which increases the economic efficiency of the process.

In the process of the present invention, a ruthenium catalyst is used which contains elemntal ruthenium on a activated carbon carrier. Principally any activated carbon material can be used as a carrier material. Examples of activated carbon include activated carbon produced from petrochemical feedstock, activated carbon from hard coal or peat, activated carbon from lignite, activated carbon of animal origin and activated carbon of plant origin, hereinafter also referred to as plant derived activated carbon. In particular, the activated carbon carrier is a plant derived activated carbon, in particular an activated carbon from coconut shell or activated carbon from wood.

Preferably, he activated carbon carrier has a BET surface of at least 500 m²/g, in particular at least 800 m²/g, and especially at least 900 m²/g, e. g. in the range of 500 to 2000 m²/g, in particular in the range of 800 to 1800 m²/g and especially in the range of 900 to 1600 m²/g. Here, the BET surface values refer to values determined in accordance with DIN ISO 9277:2022 using nitrogen physisorption.

In particular, the activated carbon carrier has a iodine number in the range of 800 to 1500, in particular in the range of 900 to 1300. The iodine number is defined as the mass of iodine in milligrams adsorbed in an aqueous solution of 1 g activated carbon when the residual iodine concentration in the filtrate is 0.02 mol/L. The iodine number of the activated carbon carrier can be determined by DIN EN 12902:2005. Typically, the activated carbon carrier essentially consists of elemental carbon. In partiuclar, the activated carbon carrier has a carbon content of at least 90% by weight, in particular at least 95% by weight, based on the total weight of the activated carbon carrier.

The activated carbon carrier can be an activated carbon powder, an activated carbon split material or a shaped body of activated carbon, e. g. strands, pellets or lobes, or activated carbon spheres. Typical powders of activated carbon have weight average particle sizes of not more than 200 µm, e. g. in the range of 5 to 200 µm, in particular in the range of 10 to 100 µm. In contrast thereto, split materials of activated carbon or shaped bodies of activated carbon have larger sizes, e. g. average diameters in the range of 500 µm to 10.000 µm, in particular in the range fom 500 µm to 5.000 µm. Here, the values of the particle size refer to the D50 value as determined by sieving in accordance with DIN 66165-1 2018.

Irrespective of the carrier form, the heterogeneous ruthenium catalyst typically generally contains 1 to 10% by weight, in particular 1.5 to 8% by weight, especially 2 to 6% by weight of ruthenium, based on the dry weight of the catalyst and calculated as elemental ruthenium.

In particular, the catalyst used in the process of the present invention, does not contain signifcant amount of metals different from ruthenium. In particular, the total amount of other metals, such as alkalimetals and earth alkaline metals, does not exceed more than 10% by weight, in particular less than 7% by weight, based on the total amount of ruthenium present in the catalyst.

The amounts of ruthenium and other metals, such as alkalimetals and transition metals, can be determined by elemental analysis, e. g. by inductively coupled plasma optical emission spectrometry (ICP-OES) using an external calibration with matrix matched standards, blank subtraction, and internal standard correction. For further details we refer to the experimental part. Here, the amounts of metal are calculated on the basis of their elemental mass.

The heterogeneous ruthenium catalyst can be produced by analogy to well known methods for producing catalysts comprising elemental ruthenium on inorganic carrier materials. These methods typically comprise the treatement of the activated carbon carrier with a ruthenium compound that can be converted into elemental ruthenium after the treatment of the activated carbon carrier material. In particular, the ruthenium compound is converted into elemental ruthenium by subjected the treated carrier material containing the ruthenium compound to a reduction step, where the ruthenium compound is converted to elemental ruthenium.

In particular, the heterogeneous ruthenium catalyst is obtainable by a process which comprises the treatment of the activated carbon carrier with an aqueous solution of a ruthenium compound, in particular with an aqueous solution of a ruthenium salt, followed by a treatment of the treated activated carbon carrier with a reducing agent, in particular with hydrogen and/or with an organic reducing agent.

Typical ruthenium compounds suitable for the treatement of the activated carbon carrier include ruthenium nitrosyl nitrate, ruthenium sulfates, such as ruthenium (III) sulfate, ruthenium halides, such as ruthenium (III) choride, and salts of ruthenic acid such as sodium ruthenate. Preferred ruthenium salts are ruthenium nitrosyl nitrate and ruthenium(III) chloride.

Preferably, the aqueous solution of the ruthenium compound used for the treatment of the activated carbon carrier has an acidic pH in order to avoid a precipitation of the ruthenium compound. In particular, the aqueous solution of the ruthenium compound has a pH of at most pH3, in particular at most pH 2. In particular the pH of the solution of the ruthenium compound used for the treatment of the activated carbon carrier has a pH in the range of 0.5 to 3, in particular in the range of 1 to 2.5. Here, the pH values refers to the values determined by a calibrated pH electrode at 22°C and ambient pressure, preferably in accordance with DIN 19268:2021. If necessary, the pH of the aqueous solution of the ruthenium compound can be adjusted to the aformenentioned ranges by means of an acid, in particular, selected from mineral acids, such as hydrochlorid acid, nitric acid or sulfuric acid.

The treatment of the activated carbon carrier material with the aqueous solution of the ruthenium compound can be carried out by analogy to techniques common to a skilled person. Typically, the activated carbon carrier is impregnated or soaked, respectively, by the aqueous solution of the ruthenium compound or immersed in the aqueous solution of the ruthenium compound or by combinations thereof. For example, the aqueous solution of the ruthenium compound can be sprayed onto a moving bed of the activated carbon carrier material, e. g. by means of a rotating drum coater, in order to ensure even distribution of the ruthenium compound on the carrier material. Alternatively, the activated carbon carrier material can be immersed or suspended in the aqueous solution of the ruthenium compound.

The concentration of the ruthenium compound and the amount of the solution are chosen such that the amount of ruthenium compound absorbed by the activated carbon carrier corresponds to the intended amount of elemental ruthenium on the activated carbon carrier in the final catalyst. The necessary amounts of the solutoin of the ruthenium compound and the concentratons can be determined by routine and estimated from the absorption capacity of the activated carbon carrier material for aqueous solutions.

The treatment of the activated carbon carrier material with the aqueous solution of the ruthenium compound is typically carried out at a temperature in the range of 20°C up to the boiling of the aqueous solution and typcially at temperatures in the range of 40 to 99 °C, especially in the range of 50 to 95°C.

As mentioned before, the treatment of the activated carbon carrier is preferably carried out with an acidic aqueous solution of the ruthenium compound, in partiuclar a ruthenium salt, to avoid a precipitation of the ruthenium compound that may cause a clogging of the ruthenium compound in the pores of the carrier material.

It has also been found to be advantageous to carry out a subsequent treatment of the treated activated carbon carrier with a base, in particular with an inorganic base, in order to fix the ruthenium compound absorbed in the pores of the activated carbon carrier material. For example, the activated carbon carrier previously treated with the acidic solution of the ruthenium compound is subsequently treated with an aqueous solution of a base, in particular an inorganic base. Alternatively, the activated carbon carrier can first be suspended in an acidic solution of the ruthenium compound and, after a certain time, e. g. after 10 to 240 minuntes, the pH value of the aqueous solution is raised to a neutral or basic pH value of at least pH 6, e. g. to pH 6 to 11 by adding a base, in particular by the additon of an aqueous solution of an inorganic base, to the suspension of the activated carbon carrier in the aqueous solution of the ruthenium compound.

Suitable bases for this purpose are in particular inorganic bases, such as alkalimetal carbononates, alkalimetal hydrogencarboates and alkalimetal hydroxides, such as sodium carbonate, sodium bicarbonate or sodium hydroxide.

The treatment with the base is typically carried out at a temperature in the range of 20°C up 110°C and typcially at temperatures in the range of 40 to 100 °C, especially in the range of 50 to 95°C.

Subsequent to the treatment of the active carbon carrier with the ruthenium compound and after the optional treatment with the base, the treated active carbon carrier is subjected to a reducing step to convert the ruthenium compound contained in the treated carrier into elemental ruthenium. In the reducing step the activated carbon carrier that has been previously treated with the ruthenium compound and optionally with a base is treated with a reducing agent suitable for reducing the ruthenium compound to elemental ruthenium. Suitable reducing agents include in particular hydrogen or organic reducing agents, in particular organic reducing agents that during the reduction of the ruthenium compound to elemental ruthenium are converted to carbon dioxide. A particular suitable organic reducing agent is selected from formic acid and the salts thereof, in particular the alkalimetal salts of formic acid, especially sodium formate.

In a preferred group of embodiments, the reducing step for obtaining the ruthenium catalyst used in the hydrogenation ethyl levulinate to gamma valerolactone comprises the reduction of the treated activated carbon carrier with an alkalimetal formate, such as sodium formate, in the presence of a base. In particular, the base is an inorganic base as mentioned above, which is in particular selected from the group consisting of alkalimetal carbononates, alkalimetal hydrogencarboates and alkalimetal hydroxides, such as sodium carbonate, sodium bicarbonate or sodium hydroxide.

In particular, the reduction with formic acid or the salt thereof is carried out at a pH of at least pH 9, more particularly at least pH 10. For this, the treated activated carbon carrier is preferably treated with an aqueous solution of formic acid or a salt thereof at a pH of at least pH 9, in particular at least pH 10, e. g. pH 9 to 13, especially pH 10 to 12. For example, the treated activated carbon carrier can be treated with an aqueous solution of an alkalimetal formate having pH of at least pH 9, in particular at least pH 10, e. g. pH 9 to 13, especially pH 10 to 12 or first with an aqueous solution of formic acid or sodium formate having preferably a pH in the range of 6 to 9 followed by a treatment under alkaline conditions, e. g. with an aqueous solution of the base at having pH of at least pH 9, in particular at least pH 10, e. g. pH 9 to 13, especially pH 10 to 12. Preferably, the reduction with an alkalimetal formate, such as sodium formate, in the presence of a base is carried out at a temperature of at least 60°C, in particular at least 80°C, e. g. in the range of 80 to 100°C. The time required for the reduction of the ruthenium compound with formic acid or a formate salt will depend on the temperature and can be determined by routine. The time required is typically in the range of 10 to 240 minutes. Typically, the treated and reduced catalyst is washed with water, in particular with deionized water to remove halogen.

In another preferred group of embodiments, the reducing step for obtaining the ruthenium catalyst used in the hydrogenation ethyl levulinate to gamma valerolactone comprises the reduction of the treated activated carbon carrier with hydrogen. Preferably, the reduction with an hydrogen is carried out by passing a stream of hydrogen of a mixture of hydrogen and nitrogen containing in particular 2 to 20 vol.-% of H₂, through the treated activated carbon carrier material at a temperature of at least 120°C, in particular at a temperature in the range of 140 to 240°C. The time required for the reduction of the ruthenium compound with hydrogen will depend on the temperature and can be determined by routine. The time required is typically in the range of 1 to 10 h.

Of course, it is also possible to reduce the ruthenium compound on the treated activated carbon carrier material by a combination of the reduction with formic acid or salt thereof and a reduction with hydrogen. In particular, the treated activated carbon carrier material can be first subjected to a reduction with formic acid or salt thereof followed by a reduction with hydrogen.

The ruthenium catalyst containing elemental ruthenium on an activated carbon carrier, in particular, the ruthenium catalyst which is obtainable by the process described herein, is particularly useful for the hydrogenation of ethyl levulinate with hydrogen to obtain gamma valerolactone.

According to the invention, the ethyl levulinate is hydrogenated with elemental hydrogen (H2) in the presence of the heterogeneous ruthenium catalyst described herein.

The hydrogenation of ethyl levulinate with hydrogen to obtain gamma valerolactone can be carried out by analogy to the hydrogenation of organic ketones with elemental hydrogen in the presence of a supported transition metal catalyst containing a transition metal on a carrier material.

To this end, the ethyl levulinate can be subjected to the hydrogenation in the form of liquid phase or gas phase, preferably in the form of liquid phase. For this, ethyl levulinate is brought into contact with the ruthenium catalyst in the presence of hydrogen.

The hydrogenation can be designed to take place either continuously or else batchwise, preference being given here to the continuous design of the process.

In a continuous process, the liquid phase, here the ethyl levulinate containing liquid stream, can by way of example be passed over a fluidized bed of ruthenium catalyst (fluidized bed method) or can be passed over a fixed bed of ruthenium catalyst (fixed bed method). In a batchwise design is also possible to carry out the hydrogenation in a suspension of the ruthenium catalyst in a liquid phase containing the ethyl levulinate, in particular in a liquid phase which essentially consists of the ethyl levulinate.

The batchwise hydrogenation can use a reaction apparatus conventionally used for this purpose, e.g. a stirred reactor.

In the process of the invention, it is preferable that the hydrogenation of ethyl levulinate is carried out in a fixed-bed reactor. For this, the hydrogenation is carried out continuously by passing the ethyl levulinate and the hydrogen through a fixed bed of the ruthenium catalyst. It is preferable that the hydrogenation of the invention is carried out continuously in fixed-bed reactors in upflow mode or in particular in downflow mode, also referred to as sump mode. The hydrogen here can be passed over the catalyst cocurrently with the ethyl levulinate to be hydrogenated, or else in countercurrent. Preferably, the hydrogen here is passed over the catalyst cocurrently with the ethyl levulinate.

Suitable apparatuses for conducting fluidized-bed-catalyst hydrogenation and fixed-bed-catalyst hydrogenation are known in the prior art, e.g. from Ullmanns Enzyklopädie der Technischen Chemie [Ullmann's Encyclopedia of Industrial Chemistry], 4th edition, volume 13, pp. 135 ff., and also from P. N. Rylander, "Hydrogenation and Dehydrogenation" in Ullmann's Encyclopedia of Industrial Chemistry, 5th edn. on CD-ROM.

Preferably, the hydrogenation of the ethyl levulinate is carried out to a degree of conversion of at least 90%, in particular at least 95%, based on the ethyl levulinate subjected to the hydrogenation.

The hydrogenation of the ethyl levulinate can be carried out in an organic solvent that is inert under the hydrogenation conditions. Suitable solvents are in particular aliphatic and alicyclic ethers, for example tetraydrofurane, dioxane, or a dialkylene glycol, such as diethylene glykol, or a mono- or dialkyl ether thereof, for example glyme, diglyme and the like. Preferably, the hydrogenation of the ethyl levulinate is carried out in the absence of organic solvents, i. e. the ethyl levulinate subjected to the hydrogenation is in essentially pure form.

Essentially in pure form is understood that the ethyl levulinate subjected to the hydrogenation has a purity of at least 95% by weight, in particular at least 97% by weight. Impurities may be e. g. water, ethanol and levulinic acid.

The hydrogenation of the ethyl levulinate is generally carried out at elevated hydrogen pressure. Preference is given to a hydrogen pressure in the range of 10 to 80 bar, in particular in the range of 15 to 70 bar, especially in the range of 20 to 60 bar.

The hydrogenation of the ethyl levulinate preferably carried out at a temperature in the range from 100 to 200°C, in particular at temperature in the range of 120 to 180°C.

Typically, the amount of the catalyst is chosen such that the amount of ruthenium is in the range of 0.02% to 2 % by weight, in particular 0.05 to 1% by weight, based on the amount of ethyl levulinate. In a continuous hydrogenation the ethyl levulinate, the catalyst load, i. e. the amount of ethyl levulinate per time and amount of catalyst is typically in the range of 20 to 1000 kg/h of ethyl levulinate per 1 kg of the ruthenium catalyst, in particular in the range of 40 to 400 kg/h of ethyl levulinate per 1 kg of the ruthenium catalyst.

The amount of hydrogen used for the hydrogenation of the ethyl levulinate is generally in the molar range 1 to 15 times of the stochiometric amount of hydrogen theoretically needed for the complete hydrogenation of the keto group in the ethyl levulinate.

Preferably, the hydrogenation is carried out in such a way that at least 90%, in particular at least 95%, especially at least 97% of the ethyl levulinate converted, i. e. is consumed in the hydrogenation reaction. The degree of conversion can be monitored by analyzing the effluent of the reactor via gas chromatography and the reaction conditions can be adjusted accordingly.

The time required for achieving the desired conversion will depend on the conditions of the hydrogenation reaction and can be established by routine. Generally, the required reaction time is in the range of 2 to 12 h, in particular in the range of 4 to 10 h. In a continuously operated reactor, the average residence time in the hydrogenation zone containing the catalyst is typically in similar ranges.

As explained above, the production of gamma valerolactone via hydrogenation of ethyl levulinate includes a hydrogenation of the keto carbonyl group of the ethyl levulinate, whereby ethyl 4-hydroxyvalerate is formed as an intermediate, which cyclizes to gamma valerolactone with formation of an ethanol as a side product.

Preferably, the process according to the invention is carried out in such a way that the ethyl 4-hydroxyvalerate formed as an intermediate makes up at most 20 mol%, in particular at most 15 mol%, based on the total amount of gamma-valerolactone and ethyl 4-hydroxyvalerate formed in the hydrogenation step. Preferably, therefore, at least 80 mol%, in particular at least 85 mol%, of the ethyl 4-hydroxyvalerate formed in the intermediate cyclize to gamma-valerolactone during the hydrogenation.

In order to further increase the yield of gamma valerolactone, the reaction mixture obtained from the hydrogenation reaction containing up to 20 mol-% of ethyl 4-hydroxyvalerate may be subjected again to a catalytic hydrogenation or to a subsequent cyclization step.

In a particular embodiment of the claimed process, a first portion P1 of the reaction mixture of the hydrogenation reaction which contains gamma valerolactone and the intermediately formed ethyl 4-hydroxyvalerate is mixed with fresh ethyl levulinate. The thus obtained mixture is then returned into the hydrogenation in the presence of the ruthenium catalyst as described herein. The remainder of the reaction mixture of the hydrogenation reaction which contains gamma valerolactone and the intermediately formed ethyl 4-hydroxyvalerate, hereinafter also referred to the second portion P2, is subjected to a workup, in particular to a distillation, in order to purify the gamma valerolactone and separate it from the ethyl 4-hydroxyvalerate. The ethyl 4-hydroxyvalerate may be subjected to a cyclization or returned into the hydrogenation reaction together with the mixture of the first portion P1 and fresh ethyl levulinate. The ratio of the first portion and the second portion, hereinafter referred to as ratio P1/P2, may vary depending on the composition of the reaction mixture of the hydrogenation reaction. Preferably the ratio P1/P2 is in the range of 80:1 to 1:2 and in particular in the range of 50:1 to 1:1 on a weight basis.

In another particular embodiment of the claimed process, a first portion P1' of the reaction mixture of the hydrogenation reaction which contains gamma valerolactone and the intermediately formed ethyl 4-hydroxyvalerate and non-reacted ethyl levulinate is mixed with fresh ethyl levulinate. The thus obtained mixture is then returned into the hydrogenation in the presence of the ruthenium catalyst as described herein. The remainder of the reaction mixture of the hydrogenation reaction which contains gamma valerolactone, the intermediately formed ethyl 4-hydroxyvalerate and non-reacted ethyl levulinate, hereinafter also referred to the second portion P2', is subjected to a hydrogenation under forced reaction conditions, in particular at higher temperature and/or higher hydrogen pressure and/or lower load to drive the hydrogenation and cyclization to higher conversion, e. g. to conversion of above 95 mol-%, based on the total amount of ethyl levulinate and ethyl 4-hydroxyvalerate in the second portion P'. The ratio of the first portion and the second portion, hereinafter referred to as ratio P1'/P2', may vary depending on the composition of the reaction mixture of the hydrogenation reaction. Preferably the ratio P1'/P2' is in the range of 80:1 to 1:2 and in particular in the range of 50:1 to 1:1 on a weight basis.

The final reaction product may be subjected to a fine distillation for increasing the purity of the gamma valerolactone.

In the following, the invention is described by way of examples.

### Examples:

### Abbreviations:

- b.w.: by weight
- cat: catalyst
- EtLev: ethyl levulinate
- EtOH: ethanol
- GC: Gas chromatography
- GVL: gamma valerolactone
- n.d.: not determined
- rpm: rotations per minute

### Analytics:

### Gas Chromatography (GC)

| | |
|---|---|
| Column: | DB-5_60/320/1 |
| Detecor: | FID (Flame ionization detector) of Shimadzu |
| Method Information: | 40°C-10min-5°C/min-130°C-20min-20°C/min-300°C-12.5min Temperature Injector/Detector: 250°C/320°C |
| | 1.5ml/min N2 Split:100:1 |
| Runtime | 69.00 |

### Determination of BET surface:

### BET was determined by in accordance with DIN ISO 9277 using the following protocol:

Samples were activated at 200°C for 12 h under vacuum. Free space was measured by He-dosing. The isotherms were measured at 77K. The pressure range for the BET fit was chosen as P/Po= 0.05-0.22. The measurements were performed using a Micromeritics ASAP2420 device.

### Elemental analysis (ruthenium):

An aliquot of the sample (0.1-0.7 g) was accurately weighed into an alumina crucible, to which 2.5 g Na₂O₂ were added and mixed with the sample. The crucible was inserted into a quartz vessel, covered a quartz lid and placed into a microwave muffle furnace. The muffle furnace program was initiated, which heated its contents stepwise to 720 °C (total heating cycle duration ca. 1 hour). After cooling down, the alumina crucible containing the digested sample material was added to a 250 mL beaker to which 30 mL concentrated HCl and ca. 60 mL H₂O were added. The beaker was coved with a watch glass and heated over a heating plate to the boiling point for ca. 30 minutes. After total dissolution of the digested mass and cooling down to room temperature, the liquid contents of the beaker were transferred and rinsed quantitatively to a 250 ml volumetric flask and the volume completed with deionized water. Each sample was prepared in duplicate. A blank sample was prepared in the same manner.

The content of Ru was determined in the thus obtained solution via inductively coupled plasma optical emission spectrometry (ICP-OES) using an external calibration with matrix matched standards, blank subtraction, and internal standard correction. The reported result was the mean of both duplicates.

### Elemental analysis (sodium):

An aliquot of the sample (0.05-0.20g) was accurately weighed into a beaker covered with a watch glass dish, to which 5 mL of H₂SO₄ were added, followed by 5 mL of HNO₃. Next, 5 mL of a mixture of H₂SO₄, HNO₃ and HClO₄ (1:2:1 by volume) were added. This last step was repeated until the resulting solution was transparent and clear in appearance. Over the course of these first acid addition steps, the beaker was heated on a hot plate in a fume hood to a final temperature of approximately 300°C, adjusting the temperature stepwise according to the respective digestive reaction taking place. Finally, all acid was steamed off by tilting the watch glass dish up to not allow anymore condensation and reflux and adjusting the temperature such that the solution was gently simmering. The resulting salts were solubilized in either HCl, HNO₃ or aqua regia depending on the analyte in question.

A blank sample was prepared in the same manner.

The content of Na was determined in the thus obtained solution via inductively coupled plasma optical emission spectrometry (ICP-OES) using an external calibration with matrix matched standards, blank subtraction, and internal standard correction. The reported result was the mean of both duplicates.

### The following activated carbon materials were used:

Activated carbon powder: Plant based acid activated carbon powder apparent density of <0.2 g/cm³, a iodine number of 1100-1200 mg/g, a methylene blue number of about 22, BET surface about 1500 m²/g and a particle size D50 of about 23 µm, as determined by laser light scattering - Commercial product: ENO Antichromos^{®} of Chemviron.

Carbon Extrudate: An activated carbon extrudate having a strand diameter of 2 mm and a BET surface about 1150 m²/g - Commercial product: Filtercarb ES2-K of Carbonitalia.

### A Production of catalysts

### Catalyst 1 (5% b.w. Ru on activated carbon powder):

500 g of demineralized water were filled 2 L glass beaker equipped with a propeller stirrer. 94 g of dry carbon powder were added to the beaker with stirring and after addition of 900 ml demineralized water the mixture was stirred for further 30 minutes. Then, a concentrated solution of ruthenium trichloride in hydrochloric acid containing 5 g ruthenium as RuCl₃ were diluted with demineralized water and the pH of the mixture was adjusted to pH 1.5 by addition of an aqueous 10% b.w. solution of sodium carbonate. The thus obtained solution of was added to the aqueous suspension of the activated carbon powder within 15 minutes with stirring and stirring of the mixture was continued for 60 minutes. Then the slurry was heated to 60-65°C and the pH was adjusted to pH 6.0-6.5 by addition of a 9.5% by weight aqueous solution of sodium bicarbonate. Then the mixture was heated to 95°C followed by the addition of a solution of 11.9 g of 85% formic acid in 40 ml water within 45 minutes and stirring at 95°C for 2 hours. Then the pH of the slurry was adjusted to pH 11-11.5 by the addition of a 30% b.w. aqueous solution of sodium hydroxide. Thereafter, cold demineralized water was added to the suspension to cool to 60-70°C. The warm solution was filtered and the solids were washed with about 3 L of cold demineralized water.

The obtained catalyst contained 4.4% by weight of ruthenium.

### Catalyst 2 (5% b.w. Ru on activated carbon extrudate):

A concentrated solution of ruthenium trichloride hydrochloric acid containing 21 g ruthenium as RuCl₃ were diluted with demineralized water to 1 L to obtain an acidic aqueous solution of ruthenium trichloride. A drum coater was filled with 375.3 g of carbon extrudate. After starting rotation of the drum the acidic aqueous solution of ruthenium trichloride was sprayed into the rotating drum within 30 minutes. Rotating was continued for 5 minutes and the thus impregnated carbon extrudate was allowed to rest for 20 minutes. A beaker was filled with 1.4 L of demineralized water, followed by adjusting the pH to 10 by addition of a 30% by weight aqueous solution of sodium hydroxide. The impregnated carbon extrudate was added to the thus prepared alkaline aqueous solution with stirring. The mixture was heated to 93°C and stirring was continued for 45 minutes while maintaining a pH of 11 by addition of a 30% by weight aqueous solution of sodium hydroxide. 116 g of sodium formate were dissolved in 200 mL of deionized water and the thus obtained solution was added within 15 minutes to the heated aqueous slurry of impregnated carbon extrudate. Heating of said mixture to 90-95°C was maintained for 30 minutes. The mixture was filtered over a Buchner funnel and the solids were washed with deionized water until filtrate was free of chloride (no visible formation of precipitate upon addition of silver nitrate).

The obtained catalyst contained 4.7% by weight of ruthenium and less than 0.3% of sodium.

For comparison, a commercial catalyst 3 containing 5% by weight of ruthenium on a zirconium oxide carrier was used.

### A Hydrogenation in autoclave (batch operation)

The hydrogenation of ethyl levulinate was carried out in a 100 ml autoclave equipped with a stirrer. 40 g ethyl levulinate and 4 g of the respective catalyst were added to the autoclave (split in the catalyst basket, powder without catalyst basket). The reaction mixture was then purged with nitrogen. Thereafter, hydrogen was pressurized to approx. 10 bar, the stirrer speed was set to 1000 rpm and the reaction mixture was heated to 180°C. After reaching the reaction temperature, hydrogen was pressurized to 40 bar and the pressure was maintained at 40 bar by adding hydrogen gas. After 6 h the heating was switched off and the autoclave was allowed to cool to 22°C. Samples were taken after 2 h, 4 h and after cooling and analyzed by GC. The results are summarized in table 1.

**Table 1:**

| Time [h] | Catalyst 1 | Catalyst 3¹⁾ |
|---|---|---|
| | Yield Valerolactone [%]²⁾ | |
| 2 | 67 | 40 |
| 4 | n.d. | 40 |
| 6 | 77 | 60 |

| | | |
|---|---|---|
| 1) not according to the invention 2) determined by GC and given as area % | | |

### A Continuous hydrogenation in tubular reactor

Ethyl levulinate was continuously fed via a heat exchanger to the top of a tubular reactor (reactor volume 289 ml, catalyst filling 100 ml) with a load of 0.4 to 0.6 kg/(h* L(catalalyst)) (feed rate of about 0.04-0.06 kg/h). Hydrogen was concurrently fed to the reactor at a pressure in the range of 40 to 60 bar. The reactor was operated in sump mode. The reactor discharge was cooled and passed through a gas-liquid separator. From the separator, part of the discharge (approx. 1000 g/h) was fed back into the reactor together with the feed via the heat exchanger and the other part was discharged into the discharge tank. The content of the discharge tank was analyzed by GC after 24 h. The detailed reaction condition and the results are summarized in table 2.

**Table 2:**

| T [°C] | p(H₂) [bar] | load¹⁾ kg/l_{cat}*h | Conversion [%] | S(GVL) ²⁾ (GVL) [%] | Yield GVL [%] | S EtOH ³⁾ [%] |
|---|---|---|---|---|---|---|
| 160 | 20 | 0.4 | 99.8 | 97.6 | 97.4 | 26.8 |
| 140 | 14 | 0.4 | 96.5 | 88.0 | 87.6 | 67.4 |

| | | | | | | |
|---|---|---|---|---|---|---|
| 1) catalyst load in kg EtLev/(L(cat)*h) 2) selectivity with respect to the formation of GVL 3) selectivity with respect to the formation of EtOH | | | | | | |

## Claims

1. A process for the production of gamma-valerlactone, which comprises the hydrogenation of ethyl levulinate with hydrogen in the presence of a heterogeneous ruthenium catalyst, which comprises elemental ruthenium supported on activated carbon carrier.

2. The process of claim 1, where the activated carbon carrier has a BET surface of at least 500 m²/g, e. g. in the range of 500 to 2000 m²/g, as determined in accordance with DIN ISO 9277 using nitrogen physisorption.

3. The process of any one of the preceding claims, where the activated carbon carrier is a plant derived activated carbon.

4. The process of any one of the preceding claims, where the heterogeneous ruthenium catalyst is obtainable by a process which comprises the treatment of the activated carbon carrier with an aqueous solution of a ruthenium salt followed by a treatment of the treated activated carbon carrier with an organic reducing agent.

5. The process of claim 4, where the organic reducing agent is formic acid or a salt thereof.

6. The process of claim 4 or 5, where the before or during the treatment with the reducing agent the treated activated carbon carrier is treated with a base.

7. The process of any one of the preceding claims, where the heterogeneous ruthenium catalyst contains 1 to 10% by weight, in particular 1.5 to 8% by weight, especially 2 to 6% by weight of ruthenium, based on the dry weight of the catalyst and calculated as elemental ruthenium.

8. The process of any one of the preceding claims, where the ethyl levulinate subjected to the hydrogenation is in essentially pure form.

9. The process of any one of the preceding claims, which is carried out in such a way that at least 90% of the ethyl levulinate subjected to the hydrogenation is consumed.

10. The process of any one of the preceding claims, comprising a hydrogenation of the ethyl levulinate and a cyclization of the intermediately formed ethyl 4-hydroxyvalerate to gamma-valerolactone, where at least 80 mol-% of the intermediately formed ethyl 4-hydroxyvalerate to gamma-valerolactone cyclizes to gamma valerolactone during the hydrogenation.

11. The process of any one of the preceding claims, where the hydrogenation is carried out such that the ethyl levulinate is in its liquid state.

12. The process of any one of the preceding claims, where the hydrogenation is carried at a temperature in the range of 100 to 200°C.

13. The process of any one of the preceding claims, where the hydrogenation is carried at a hydrogen pressure in the range of 10 to 60 bar.

14. The process of any one of the preceding claims, where the hydrogenation is carried out continuously by passing the ethyl levulinate and the hydrogen through a fixed bed of the ruthenium catalyst.
